# EUROPEAN PATENT SPECIFICATION

(11) **EP 2 553 446 B1**
(45) Date of publication and mention of the grant of the patent: **06.01.2016**
(21) Application number: 11710884.5
(22) Date of filing: 24.02.2011
(51) Int. Cl.: G01N 33/487, G01N 11/00

(54) **COPD EXACERBATION DETECTION USING SPUTUM ANALYSIS**
NACHWEIS VON COPD-VERSCHLIMMERUNG MITTELS SPEICHELANALYSE
DÉTECTION D'UNE EXACERBATION DE LA MALADIE PULMONAIRE OBSTRUCTIVE CHRONIQUE (COPD) À L'AIDE D'UNE ANALYSE DES EXPECTORATIONS

(30) Priority: 29.03.2010 US 318492 P
(43) Date of publication of application: 06.02.2013
(73) Proprietor: Koninklijke Philips N.V., 5656 AE Eindhoven (NL)
(72) Inventor: ATAKHORRAMI, Maryam, Briarcliff Manor New York 10510-8001 (US); CHEUNG, Amy, Oi Mee, Briarcliff Manor, New York 10510-8001 (US)
(74) Representative: van Velzen, Maaike Mathilde
(86) International application number: PCT/IB2011/050788
(87) International publication number: WO 2011/121462

(56) References cited:
- US-A1- 2005 164 334
- SERISIER DAVID J ET AL: "Macrorheology of cystic fibrosis, chronic obstructive pulmonary disease & normal sputum", RESPIRATORY RESEARCH, BIOMED CENTRAL LTD., LONDON, GB, vol. 10, no. 1, 6 July 2009 (2009-07-06), page 63, XP021055543, ISSN: 1465-9921, DOI: DOI:10.1186/1465-9921-10-63
- Lewis, Keir e.: "Applying new technologies in chronic obstructive pulmonary disease (COPD)", , 9 February 2010 (2010-02-09), XP002643749, Retrieved from the Internet: URL:http://www.controlled-trials.com/ISRCT N82911859 [retrieved on 2011-06-21]
- DULFANO ET AL: "Sputum viscoelasticity in chronic bronchitis", THE AMERICAN REVIEW OF RESPIRATORY DISEASE, AMERICAN THORACIC SOCIETY, US, vol. 104, no. 1, 1 July 1971 (1971-07-01), pages 88-98, XP008138137, ISSN: 0003-0805
- CLAUS VOGELMEIER; BUHL R; CRIÉE C P; GILLISSEN A; KARDOS P; KÖHLER D; ET AL.: "Leitlinie der Deutschen Atemwegsliga und der Deutschen Gesellschaft für Pneumologie und Beatmungsmedizin zur Diagnostik und Therapie von Patienten mit chronisch obstruktiver Bronchitis und Lungenemphysem (COPD)", PNEUMOLOGIE, vol. 61, 13 April 2007 (2007-04-13), pages E1-E40, XP055001054, DOI: 10.1055/s-2007-959200
- LAI S K ET AL: "Micro- and macrorheology of mucus", ADVANCED DRUG DELIVERY REVIEWS, ELSEVIER, AMSTERDAM, NL, vol. 61, no. 2, 27 February 2009 (2009-02-27), pages 86-100, XP025950267, ISSN: 0169-409X, DOI: 10.1016/J.ADDR.2008.09.012 [retrieved on 2009-01-03]

## Description

The present invention relates to the diagnosis and/or monitoring of chronic obstructive pulmonary disease in subjects through viscoelastic properties of sputum.

Chronic obstructive pulmonary disease (COPD) is a respiratory disease that is characterized by inflammation of the airways. It is characterized by an airflow limitation that is not fully reversible. The airflow limitation is both progressive and associated with an abnormal inflammatory response of the lungs to noxious particles or gases. Symptoms of COPD may include coughing, wheezing and the production of mucus and the degree of severity can, in part, be viewed in terms of the volume and color of secretions.

Exacerbations are the worsening of COPD symptoms. The exacerbations may be associated with a variable degree of physiological deterioration. The exacerbations may be characterized by increased coughing, dyspnea (i.e., shortness of breath) and production of sputum. Typically, exacerbations are detected by a general practitioner or a hospital physician. In clinical trials, questionnaires are used to identify the occurrence of an exacerbation.

The exacerbations are normally caused by viral or bacterial infections and, depending on the severity, may lead to hospitalization of the COPD patients. The frequency of exacerbations increases during the winter months due to cold stresses on the patient's body. This may be due to a combination of a) the cooling of facial skin and airways resulting in broncho constriction, and b) the thermoregulatory system becoming less effective with age, thus making COPD patients more susceptible for respiratory infections.

The exacerbations not only limit the performance of daily activities, but also significantly decrease the health related quality of life of COPD patients. A high frequency of exacerbations is linked to a poor prognosis for survival. Also, the exacerbations often may result in hospitalization, which is the main determinant of the overall healthcare expenditure for COPD patients.

Accordingly, it is an object of the present invention to provide a COPD exacerbation detection technique that overcomes the shortcomings of conventional methodologies. This object is achieved according to one embodiment of the present invention by providing a system configured to diagnose and/or monitor COPD. In one embodiment the system comprises a measurement appliance and one or more processors configured to execute computer program modules. The measurement appliance is configured to generate an output signal indicating information related to the elasticity and viscosity of sputum of a subject. The computer program modules comprise a viscosity module, an elasticity module, and a diagnosis/monitoring module. The viscosity module is configured to determine a value of a first modulus for the sputum of the subject based on the output signal of the measurement appliance, wherein the first modulus quantifies resistance to the tendency to flow. The elasticity module is configured to determine a value of a second modulus for the sputum of the subject based on the output signal of the subject, wherein the second modulus quantifies tendency to recover original shape following stress induced deformation. The diagnosis/monitoring module is configured to diagnose and/or monitor COPD in the subject based on both the value of the first modulus and the value of the second modulus.

Another aspect of the invention relates to a method of diagnosing and/or monitoring COPD. In one embodiment, the method comprises determining a value of a first modulus for sputum of a subject, wherein the first modulus quantifies resistance to the tendency to flow; determining a value of a second modulus for the sputum of the subject, wherein the second modulus quantifies tendency to recover original shape following stress induced deformation; and diagnosing and/or monitoring COPD in the subject based on both the value of the first modulus and the value of the second modulus.

Yet another aspect of the invention relates to a system configured to diagnose and/or monitor COPD. In one embodiment, the system comprises means for determining a value of a first modulus for sputum of a subject, wherein the first modulus quantifies resistance to the tendency to flow; means for determining a value of a second modulus for the sputum of the subject, wherein the second modulus quantifies tendency to recover original shape following stress induced deformation; and means for diagnosing and/or monitoring COPD in the subject based on both the value of the first modulus and the value of the second modulus.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. In one embodiment of the invention, the structural components illustrated herein are drawn to scale. It is to be expressly understood, however, that the drawings are for the purpose of illustration and description only and are not a limitation of the invention. In addition, it should be appreciated that structural features shown or described in any one embodiment herein can be used in other embodiments as well. It is to be expressly understood, however, that the drawings are for the purpose of illustration and description only and are not intended as a definition of the limits of the invention.
FIG. 1 illustrates a system configured to diagnose and/or monitor chronic obstructive pulmonary disease in a subject, according to one or more embodiments of the invention;
FIG. 2 illustrates the impact of COPD on the viscosity of sputum;
FIG. 3 illustrates the impact of COPD on elasticity of sputum; and
FIG. 4 illustrates a method of diagnosing and/or monitoring COPD in a subject, in accordance with one or more embodiments of the invention.

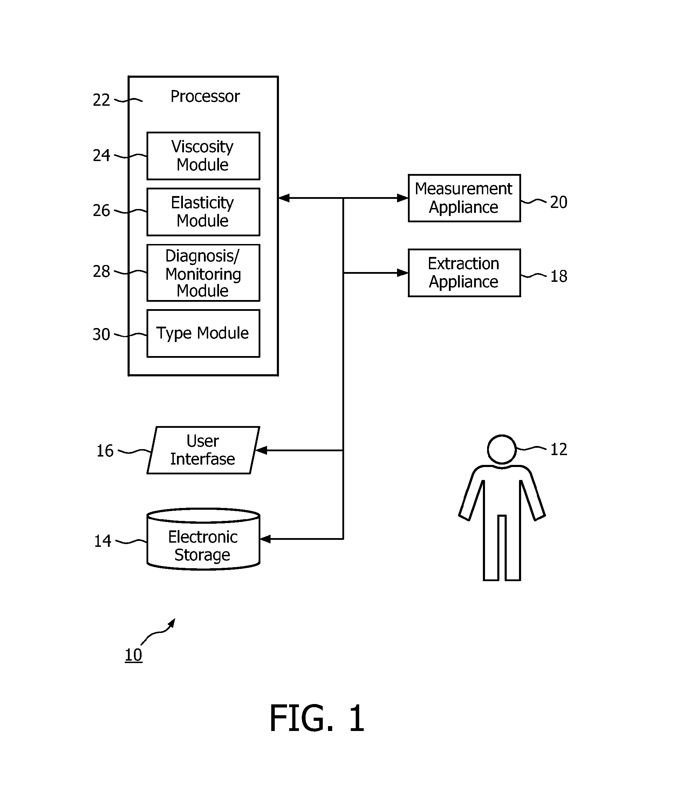

FIG. 1 illustrates a system 10 configured to diagnose and/or monitor chronic obstructive pulmonary disease in a subject 12. System 10 may provide enhanced diagnosis and/or monitoring of COPD with respect to conventional systems. For example, system 10 may diagnose and/or monitor COPD with an enhanced accuracy, may detect onset of COPD earlier on, may discern between different types of COPD with enhanced accuracy, and/or provide other enhancements. In one embodiment, system 10 includes electronic storage 14, a user interface 16, an extraction appliance 18, a measurement appliance 20, a processor 22, and/or other components.

In one embodiment, electronic storage 14 comprises electronic storage media that electronically stores information. The electronic storage media of electronic storage 14 may include one or both of system storage that is provided integrally (i.e., substantially non-removable) with system 10 and/or removable storage that is removably connectable to system 10 via, for example, a port (e.g., a USB port, a firewire port, etc.) or a drive (e.g., a disk drive, etc.). Electronic storage 14 may include one or more of optically readable storage media (e.g., optical disks, etc.), magnetically readable storage media (e.g., magnetic tape, magnetic hard drive, floppy drive, etc.), electrical charge-based storage media (e.g., EEPROM, RAM, etc.), solid-state storage media (e.g., flash drive, etc.), and/or other electronically readable storage media. Electronic storage 14 may store software algorithms, information determined by processor 22, information received via user interface 16, and/or other information that enables system 10 to function properly. Electronic storage 14 may be a separate component within system 10, or electronic storage 14 may be provided integrally with one or more other components of system 10 (e.g., processor 22).

User interface 16 is configured to provide an interface between system 10 and a user (e.g., subject 12, a caregiver, a researcher, a therapy decision-maker, etc.) through which the user may provide information to and receive information from system 10. This enables data, results, and/or instructions and any other communicable items, collectively referred to as "information," to be communicated between the user and system 10. Examples of interface devices suitable for inclusion in user interface 16 include a keypad, buttons, switches, a keyboard, knobs, levers, a display screen, a touch screen, speakers, a microphone, an indicator light, an audible alarm, and a printer. In one embodiment, the functionality of which is discussed further below, user interface 16 actually includes a plurality of separate interfaces. For example, one interface may be provided for subject 12, and a separate interface may be provided to another user (e.g., caregiver, researcher, therapy decision-maker, etc.). The interface provided to the other user may be provided remotely to subject 12.

It is to be understood that other communication techniques, either hardwired or wireless, are also contemplated by the present invention as user interface 16. For example, the present invention contemplates that user interface 16 may be integrated with a removable storage interface provided by electronic storage 14. In this example, information may be loaded into system 10 from removable storage (e.g., a smart card, a flash drive, a removable disk, etc.) that enables the user(s) to customize the implementation of system 10. Similarly, information about subject 12 may be stored to removable storage, and then accessed remotely from the removable storage by another user. Other exemplary input devices and techniques adapted for use with system 10 as user interface 14 include, but are not limited to, an RS-232 port, RF link, an IR link, modem (telephone, cable or other). In short, any technique for communicating information with system 10 is contemplated by the present invention as user interface 14.

Extraction appliance 18 is configured to obtain sputum from subject 12. In one embodiment, this includes clearing secretions from the airway of subject 12. The extraction appliance 18 may include one or more of any appliance that extracts secretions from the airway of subject 12. For example, extraction appliance 18 may include a FLUTTER® mucus removal device, a cough assist mucus removal device, and/or other appliances suitable for obtaining sputum from subject 12.

It will be appreciated that sputum may be produced manually by subject 12 without assistance from extraction appliance 18. As such, in one embodiment, extraction appliance 18 may simply be a receptacle into which subject 12 deposits manually produced sputum.

Measurement appliance 20 is configured to generate an output signal indicating information related to both elasticity and viscosity of sputum of subject 12. In one embodiment, measurement appliance 20 comprises a viscometer, a rheometer, and/or other devices, apparatuses, and/or appliances configured to measure elasticity and/or viscosity. Although measurement appliance 20 is illustrated in FIG. 1 as a single appliance, this is not intended to be limiting. It will be appreciated that separate appliances may be implemented to measure elasticity and viscosity separately.

Processor 22 is configured to provide information processing capabilities in system 10. As such, processor 22 may include one or more of a digital processor, an analog processor, a digital circuit designed to process information, an analog circuit designed to process information, a state machine, and/or other mechanisms for electronically processing information. Although processor 22 is shown in FIG. 1 as a single entity, this is for illustrative purposes only. In some implementations, processor 22 may include a plurality of processing units. These processing units may be physically located within the same device, or processor 22 may represent processing functionality of a plurality of devices operating in coordination. For example, in one embodiment, the functionality attributed below to processor 22 is divided between a first processor that is operatively connected to extraction appliance 18 in a first device that is local to subject 12, and a second processor that communicates with the first device (e.g., over a communication line, over a network, through transferable storage media, etc.) to obtain information generated by the first processor and further process the obtained information.

As is shown in FIG. 1, processor 22 may be configured to execute one or more computer program modules. The one or more computer program modules may include one or more of a viscosity module 24, an elasticity module 26, a diagnosis/monitoring module 28, a type module 30, and/or other modules. Processor 22 may be configured to execute modules 24, 26, 28, and/or 28 by software; hardware; firmware; some combination of software, hardware, and/or firmware; and/or other mechanisms for configuring processing capabilities on processor 22.

It should be appreciated that although modules 24, 26, 28, and 28 are illustrated in FIG. 1 as being co-located within a single processing unit, in implementations in which processor 38 includes multiple processing units, one or more of modules 24, 26, 28, and/or 28 be located remotely from the other modules. The description of the functionality provided by the different modules 24, 26, 28, and/or 28 described below is for illustrative purposes, and is not intended to be limiting, as any of modules 24, 26, 28, and/or 28 may provide more or less functionality than is described. For example, one or more of modules 24, 26, 28, and/or 28 may be eliminated, and some or all of its functionality may be provided by other ones of modules 24, 26, 28, and/or 28. As another example, processor 22 may be configured to execute one or more additional modules that may perform some or all of the functionality attributed below to one of modules 24, 26, 28, and/or 28.

Viscosity module 24 is configured to quantify the viscosity of sputum from subject 12. Viscosity refers to resistance to tendency to flow. The viscosity module 24 quantifies the viscosity of sputum from subject 12 based on output signals generated by measurement appliance 20. For example, viscosity module 24 may determine, based on the output signals generated by measurement appliance 20, a value of a first modulus (e.g., a viscose or loss modulus) that quantifies the viscosity of the sputum.

Elasticity module 26 is configured to quantify the elasticity of sputum from subject 12. Elasticity refers to tendency to recover original shape following stress induced deformation. Elasticity module 26 quantifies the elasticity of sputum from subject 12 based on output signals generated by measurement appliance 20. For example, elasticity module 26 may determine, based on the output signals generated by measurement appliance 20, a value of a second modulus (e.g., an elastic or storage modulus) that quantifies the elasticity of the sputum.

In one embodiment, information determined by elasticity module 26 and diagnosis/monitoring module 28 is provided to a user via user interface 16 without further processing. This may enable manual performance of further diagnosis and/or monitoring of COPD in subject 12 based on the information determined by elasticity module 26 and/or diagnosis/monitoring module 28. For example, the diagnosis and/or monitoring of COPD may be performed in accordance with the principles described below.

Diagnosis/monitoring module 28 is configured to diagnose and/or monitor COPD in subject 12 based on both of the viscosity and the elasticity of sputum from subject 12. This includes diagnosing and/or monitoring COPD in subject 12 based on the first modulus, as determined by viscosity module 24, and on the second modulus, as determined by elasticity module 26.

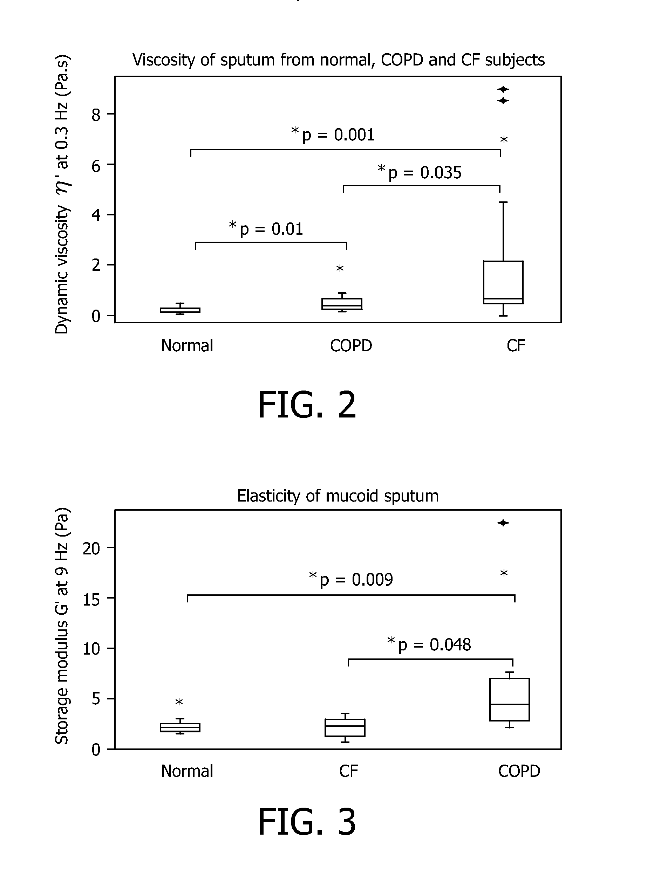

Indicators of COPD onset and/or exacerbation include increases in viscosity and elasticity of sputum. For example, FIGS. 2 and 3 illustrate the impact of COPD on sputum viscosity and elasticity, respectively, with respect to normal subjects and subjects suffering from cystic fibrosis, which is known from Serisier et al., Macrorheology of cystic fibrosis, chronic obstructive pulmonary disease & normal sputum, Respiratory Research 2009, 10:63 (doi:10.1186/1465-9921-10-63).

As can be seen in FIG. 2, the viscosity of sputum in subjects suffering onset and/or exacerbation of COPD is slightly elevated with respect to normal subjects and subjects suffering from cystic fibrosis. FIG. 3 shows how elasticity of sputum is also elevated with respect to normal subjects and/or subjects suffering from cystic fibrosis.

Returning to FIG. 1, in one embodiment, diagnosis/monitoring module 28 is configured to obtain the determinations of the first modulus and the second modulus from viscosity module 24 and elasticity module 26, respectively, and to identify onset and/or exacerbation of COPD in subject 12 based on the relationships illustrated in FIGS. 2 and 3. Such monitoring and/or diagnosis may include determining if the first modulus and the second modulus each breach threshold values corresponding to onset and/or exacerbation of COPD. These thresholds may be fixed thresholds, or may be determined based on baseline levels of elasticity and/or viscosity previously determined for subject 12.

In one embodiment, rather than comparing the values of the first modulus and the second modulus to separate thresholds, diagnosis/monitoring module 28 may identify onset and/or exacerbation of COPD based on a single determination that includes both the first modulus and the second modulus. For example, diagnosis/monitoring module 28 may determine a risk metric representing the risk of COPD onset or exacerbation. The risk metric may be determined according to a function describing the risk metric as a function of both the first modulus and the second modulus.

By identifying onset and/or exacerbation of COPD based on the first modulus and the second modulus, diagnosis/monitoring module 28 may provide a warning of onset or exacerbation of COPD earlier than other diagnostic techniques or systems, may provide a more accurate warning of onset or exacerbation of COPD than other early diagnosis techniques or systems, and/or may provide other enhancements.

Identifications of onset and/or exacerbation of COPD by diagnosis/monitoring module 28 may be communicated to one or more users through user interface 16. For example, user interface 16 may provide an indication to subject 12 of onset and/or exacerbation of COPD. In one embodiment, an indication may be provided to one or more users other than subject 12. For example, user interface 16 may provide the indication to a caregiver, a researcher, a therapy decision-maker, and/or other users.

In one embodiment, upon identification of onset and/or exacerbation of COPD in subject 12, treatment of subject 12 may commence. For example, to treat bacterial COPD, antibiotics may be prescribed. The diagnosis/monitoring module 28 may then provide information about the effectiveness of the treatment. For example, if treatment is effective, then the first modulus, the second modulus, and/or a risk metric determined from the first modulus and/or the second modulus will indicate the effectiveness. If, on the other hand, treatment is not effective, then the first modulus, the second modulus, and/or the risk metric will indicate the ineffectiveness of treatment.

Type module 30 is configured to determine a type of COPD in subject 12. The types that may be identified by type module 30 may include one or more of bacterial COPD, viral COPD, and/or inflammatory COPD. The type module 30 is configured to determine the type of COPD based on differences in the impact these different types of COPD have on elasticity and/or viscosity of the sputum of subject 12. By way of nonlimiting example, bacterial COPD tends to increase elasticity of sputum faster and/or to a further extent than viral COPD. Thus, upon identification of an onset or exacerbation of COPD in subject 12 by diagnosis/monitoring module 28, type module 30 may analyze the first and/or the second modulus determined by viscosity module 24 and/or elasticity module 26 to determine whether the elasticity of the sputum of subject 12 indicates the onset of exacerbation is of bacterial or viral COPD. For example, based on an increase in the value of the second modulus by about 25% or greater over a baseline value (e.g., a previously measured value for subject 12), type module 30 may determine that an onset of COPD is of a bacterial type.

It will be appreciated that distinguishing between viral and bacterial chronic obstructive pulmonary disease is significant at least because the treatments for these two types of COPD are different. Bacterial COPD is generally treated with antibiotics, while antibiotics would not have a substantial impact on viral COPD. In fact, taking antibiotics while suffering from viral COPD could actually have a negative effect (e.g., antibiotic resistance).

In one embodiment, the determination of the type of chronic obstructive pulmonary disease made by type module 30 may be further based on other techniques for distinguishing between the types of COPD. For example, the determination of the type of COPD may be further based on the color of the sputum obtained from subject 12, and/or through other techniques for distinguishing between the types of COPD.

Determinations of the type of COPD made by type module 30 may be provided to one or more users through user interface 16. For example, user interface 16 may provide an indication to subject 12 of the type of COPD. In one embodiment, an indication of the type of COPD may be provided to one or more users other than subject 12. For example, user interface 16 may provide the indication to a caregiver, a researcher, a therapy decision-maker, and/or other users.

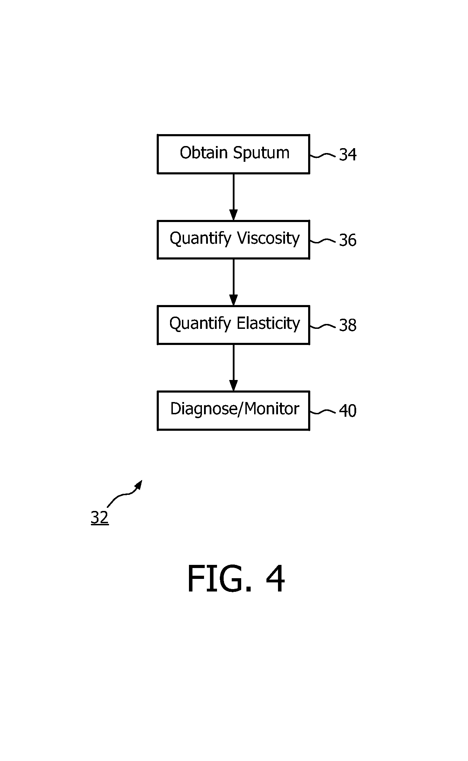

FIG. 4 illustrates a method 32 of diagnosing and/or monitoring chronic obstructive pulmonary disease in a subject. The operations of method 32 presented below are intended to be illustrative. In some embodiments, method 32 may be accomplished with one or more additional operations not described, and/or without one or more of the operations discussed. Additionally, the order in which the operations of method 32 are illustrated in FIG. 4 and described below is not intended to be limiting.

In some embodiments, method 32 may be implemented in one or more processing devices (e.g., a digital processor, an analog processor, a digital circuit designed to process information, an analog circuit designed to process information, a state machine, and/or other mechanisms for electronically processing information). The one or more processing devices may include one or more devices executing some or all of the operations of method 32 in response to instructions stored electronically on an electronic storage medium. The one or more processing devices may include one or more devices configured through hardware, firmware, and/or software to be specifically designed for execution of one or more of the operations of method 32. It will be appreciated that not all of the operations are performed by the one or more processing devices. In one embodiment, one or more of the operations are performed manually by a user.

At an operation 34, sputum is obtained from the subject. The sputum may be obtained manually, by an extraction appliance similar to or the same as extraction appliance 18 (shown in FIG. 1 and described above), and/or by some other mechanism.

At an operation 36, viscosity of the sputum obtained at operation 34 is quantified. This may include determining a value of a first modulus that quantifies viscosity. In one embodiment, operation 36 is performed by a measurement appliance and viscosity module similar to or the same as measurement appliance 20 and viscosity module 24 (shown in FIG. 1 and described above).

At an operation 38, elasticity of the sputum obtained at operation 34 is quantified. This may include determining a value of a second modulus that quantifies elasticity. In one embodiment, operation 38 is performed by a measurement appliance and elasticity module similar to or the same as measurement appliance 20 and elasticity module 26 (shown in FIG. 1 and described above).

At an operation 40, COPD in the subject is diagnosed and/or monitored based on the viscosity and the elasticity of the sputum obtained at operation 34. The diagnosis and/or monitoring is performed based on the first and second modulus determined at operations 36 and 38, respectively. The diagnosis and/or monitoring may include one or more of identifying onset and/or exacerbation of COPD, monitoring the effectiveness of treatment of COPD, determining a type of COPD, and/or other diagnosis and/or monitoring. In one embodiment, operation 40 is performed by a diagnosis/monitoring module and/or a type module similar to or the same as diagnosis/monitoring module 28 and/or type module 30 (shown in FIG. 1 and described above).

Because of the damage done when an exacerbation takes place, it can be appreciated that the present invention provides the ability to detect the onset of an exacerbation early and to initiate treatments aimed at reducing the damage caused by the exacerbation. As such, early detection and effective treatment of COPD exacerbation may help COPD patients lead an improved quality of life and may lower the healthcare costs for COPD patients.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

## Claims

1. A system (10) configured to diagnose and/or monitor chronic obstructive pulmonary disease, the system comprising:
means (24) for determining a value of a first modulus for sputum of a subject, wherein the first modulus quantifies resistance to the tendency to flow;
means (26) for determining a value of a second modulus for the sputum of the subject, wherein the second modulus quantifies tendency to recover original shape following stress induced deformation; and
means (28) for diagnosing and/or monitoring chronic obstructive pulmonary disease in the subject based on both the value of the first modulus and the value of the second modulus, wherein said means (28) for diagnosing and/or monitoring comprises means (30) for identifying an onset and/or an exacerbation of chronic obstructive pulmonary disease in the subject based on the first modulus and/or the second modulus.

2. The system of claim 1, wherein the system comprises:
(a) a measurement appliance (20) configured to generate an output signal indicating information related to the elasticity and viscosity of sputum of a subject; and
(b) a processor (22) configured to execute computer program modules, the computer program modules comprising a viscosity module (24), an elasticity module (26), a diagnosis/monitoring module (28), wherein:
the means (24) for determining the value of the first modulus comprises the viscosity module (24);
the means (26) for determining the value of the second modulus comprises the elasticity module (26); and
the means (28) for diagnosing and/or monitoring chronic obstructive pulmonary disease in the subject further comprises the diagnosis/monitoring module.

3. The system of claim 1, wherein the means (28) for diagnosing and/or monitoring comprises means (30) for identifying the onset of chronic obstructive pulmonary disease in the subject based on both the value of the first modulus and the value of the second modulus.

4. The system of any one of claims 1 to 3, wherein the means (30) for identifying the onset and/or the exacerbation of chronic obstructive pulmonary disease in the subject is arranged to identify bacterial chronic obstructive pulmonary disease based on an increase in the second modulus.

5. The system of claim 1, wherein the means (28) for diagnosing and/or monitoring comprises means for assessing an effectiveness of a treatment of chronic obstructive pulmonary disease in the subject based on both the value of the first modulus and the value of the second modulus.

6. An in vitro method of diagnosing and/or monitoring chronic obstructive pulmonary disease, the method comprising:
determining a value of a first modulus for sputum of a subject, wherein the first modulus quantifies resistance to the tendency to flow;
determining a value of a second modulus for the sputum of the subject, wherein the second modulus quantifies tendency to recover original shape following stress induced deformation; and
diagnosing and/or monitoring chronic obstructive pulmonary disease in the subject based on both the value of the first modulus and the value of the second modulus, wherein the diagnosing and/or monitoring comprises identifying an onset and/or an exacerbation of chronic obstructive pulmonary disease in the subject based on the first modulus and/or the second modulus.

7. The method of claim 6, wherein the diagnosing and/or monitoring comprises identifying the onset of chronic obstructive pulmonary disease in the subject based on both the value of the first modulus and the value of the second modulus.

8. The method of claim 6 or 7, wherein the identifying the onset and/or exacerbation of chronic obstructive pulmonary disease in the subject includes identifying bacterial chronic obstructive pulmonary disease based on an increase in the second modulus.

9. The method of claim 6, wherein the diagnosing and/or monitoring comprises assessing an effectiveness of a treatment of chronic obstructive pulmonary disease in the subject based on both the value of the first modulus and the value of the second modulus.

## Patentansprüche

1. System (10), das dafür eingerichtet ist, chronisch obstruktive Lungenkrankheit zu diagnostizieren und/oder zu überwachen, wobei das System Folgendes umfasst:
Mittel (24) zum Ermitteln eines Werts eines ersten Moduls für Speichel einer Person, wobei das Modul den Widerstand gegen eine Fließtendenz quantifiziert;
Mittel (26) zum Ermitteln eines Wertes eines zweiten Moduls für den Speichel der Person, wobei das zweite Modul die Tendenz quantifiziert, nach einer durch Beanspruchung verursachten Verformung wieder zur Originalform zurückzukehren; und
Mittel (28) zum Diagnostizieren und/oder Überwachen der chronisch obstruktiven Lungenkrankheit bei der Person basierend sowohl auf dem Wert des ersten Moduls als auch auf dem Wert des zweiten Moduls, wobei das genannte Mittel (28) zum Diagnostizieren und/oder Überwachen Mittel (30) zum Identifizieren eines Ausbruchs und/oder einer Verschlimmerung der chronisch obstruktiven Lungenkrankheit bei der Persson basierend auf dem ersten Modul und/oder dem zweiten Modul umfasst.

2. System nach Anspruch 1, wobei das System Folgendes umfasst:
(a) ein Messgerät (20), das dafür eingerichtet ist, ein Ausgangssignal zu erzeugen, das Informationen in Bezug auf die Elastizität und Viskosität des Speichels einer Person angibt; und
(b) einen Prozessor (22), der dafür eingerichtet ist, Computerprogrammelemente auszuführen, wobei die Computerprogrammelemente ein Viskositätselement (24), ein Elastizitätselement (26), ein Diagnose-/Überwachungselement (28) umfassen, wobei:
das Mittel (24) zum Ermitteln des Werts des ersten Moduls das Viskositätselement (24) umfasst;
das Mittel (26) zum Ermitteln des Werts des zweiten Moduls das Elastizitätselement (26) umfasst; und
das Mittel (28) zum Diagnostizieren und/oder Überwachen der chronisch obstruktiven Lungenkrankheit bei der Person ferner das Diagnose-/Überwachungselement umfasst.

3. System nach Anspruch 1, wobei das Mittel (28) zum Diagnostizieren und/oder Überwachen Mittel (30) zum Identifizieren des Ausbruchs der chronisch obstruktiven Lungenkrankheit bei der Person basierend auf sowohl dem Wert des ersten Moduls als auch dem Wert des zweiten Modus umfasst.

4. System nach einem der Ansprüche 1 bis 3, wobei das Mittel (30) zum Identifizieren des Ausbruchs und/oder der Verschlimmerung der chronisch obstruktiven Lungenkrankheit bei der Person dafür ausgelegt ist, die bakterielle chronisch obstruktive Lungenkrankheit basierend auf einer Zunahme im zweiten Modul zu identifizieren.

5. System nach Anspruch 1, wobei das Mittel (28) zum Diagnostizieren und/oder Überwachen Mittel zum Bewerten einer Wirksamkeit einer Behandlung der chronisch obstruktiven Lungenkrankheit bei der Person basierend auf sowohl dem Wert des ersten Moduls als auch dem Wert des zweiten Moduls umfasst.

6. In-vitro-Verfahren zum Diagnostizieren und/oder Überwachen von chronisch obstruktiver Lungenkrankheit, wobei das Verfahren Folgendes umfasst:
Ermitteln eines Werts eines ersten Moduls für Speichel einer Person, wobei das Modul den Widerstand gegen eine Fließtendenz quantifiziert;
Ermitteln eines Wertes eines zweiten Moduls für den Speichel der Person, wobei das zweite Modul die Tendenz quantifiziert, nach einer durch Beanspruchung verursachten Verformung wieder zur Originalform zurückzukehren; und
Diagnostizieren und/oder Überwachen der chronisch obstruktiven Lungenkrankheit bei der Person basierend sowohl auf dem Wert des ersten Moduls als auch auf dem Wert des zweiten Moduls, wobei das Diagnostizieren und/oder Überwachen das Identifizieren eines Ausbruchs und/oder einer Verschlimmerung der chronisch obstruktiven Lungenkrankheit bei der Person basierend auf dem ersten Modul und/oder dem zweiten Modul umfasst.

7. Verfahren nach Anspruch 6, wobei das Diagnostizieren und/oder Überwachen das Identifizieren des Ausbruchs der chronisch obstruktiven Lungenkrankheit bei der Person basierend auf sowohl dem Wert des ersten Moduls als auch dem Wert des zweiten Moduls umfasst.

8. Verfahren nach Anspruch 6 oder 7, wobei das Identifizieren des Ausbruchs und/oder der Verschlimmerung der chronisch obstruktiven Lungenkrankheit bei der Person das Identifizieren von bakterieller chronisch obstruktiver Lungenkrankheit basierend auf einer Zunahme im zweiten Modul umfasst.

9. Verfahren nach Anspruch 6, wobei das Diagnostizieren und/oder Überwachen das Bewerten einer Wirksamkeit einer Behandlung der chronisch obstruktiven Lungenkrankheit bei der Person basierend auf sowohl dem Wert des ersten Moduls als auch dem Wert des zweiten Moduls umfasst.

## Revendications

1. Système (10) conçu pour diagnostiquer et/surveiller une bronchopneumopathie obstructive chronique, le système comprenant :
un moyen (24) servant à déterminer une valeur d'un premier module pour l'expectoration d'un sujet, dans lequel le premier module quantifie la résistance à la tendance à l'écoulement ;
un moyen (26) servant à déterminer une valeur d'un second module pour l'expectoration du sujet, dans lequel le second module quantifie la tendance à la récupération d'une forme d'origine après déformation induite par une contrainte ; et
un moyen (28) servant à diagnostiquer et/ou à surveiller une bronchopneumopathie obstructive chronique chez le sujet sur la base à la fois de la valeur du premier module et de la valeur du second module, dans lequel ledit moyen (28) de diagnostic et/ou de surveillance comprend un moyen (30) permettant d'identifier le début et/ou l'exacerbation d'une bronchopneumopathie obstructive chronique chez le sujet sur la base du premier module et/ou du second module.

2. Système selon la revendication 1, dans lequel le système comprend :
(a) un appareil de mesure (20) conçu pour générer un signal de sortie indiquant des informations associées à l'élasticité et à la viscosité de l'expectoration d'un sujet ; et
(b) un processeur (22) configuré pour exécuter des modules de programme informatique, les modules de programme informatique comprenant un module de viscosité (24), un module d'élasticité (26), un module de diagnostic/surveillance (28), dans lequel :
le moyen (24) servant à déterminer la valeur du premier module comprend le module de viscosité (24) ;
le moyen (26) servant à déterminer la valeur d'un second module comprend le module d'élasticité (26) ; et
le moyen (28) servant à diagnostiquer et/ou à surveiller une bronchopneumopathie obstructive chronique chez le sujet comprend en outre le module de diagnostic/surveillance.

3. Système selon la revendication 1, dans lequel le moyen (28) de diagnostic et/ou de surveillance comprend un moyen (30) permettant d'identifier le début de la bronchopneumopathie obstructive chronique chez le sujet sur la base à la fois de la valeur du premier module et de la valeur du second module.

4. Système selon l'une quelconque des revendications 1 à 3, dans lequel le moyen (30) permettant d'identifier le début et/ou l'exacerbation de la bronchopneumopathie obstructive chronique chez le sujet sert à identifier une bronchopneumopathie obstructive chronique d'origine bactérienne sur la base d'une augmentation du second module.

5. Système selon la revendication 1, dans lequel le moyen (28) de diagnostic et/ou de surveillance comprend un moyen permettant d'évaluer l'efficacité d'un traitement de la bronchopneumopathie obstructive chronique chez le sujet sur la base à la fois de la valeur du premier module et de la valeur du second module.

6. Procédé in vitro de diagnostic et/ou de surveillance d'une bronchopneumopathie obstructive chronique, le procédé comprenant :
la détermination d'une valeur d'un premier module pour l'expectoration d'un sujet, dans lequel le premier module quantifie la résistance à la tendance à l'écoulement ;
la détermination d'une valeur d'un second module pour l'expectoration du sujet, dans lequel le second module quantifie la tendance à la récupération d'une forme d'origine après déformation induite par une contrainte ; et
le diagnostic et/ou la surveillance d'une bronchopneumopathie obstructive chronique chez le sujet sur la base à la fois de la valeur du premier module et de la valeur du second module, dans lequel le diagnostic et/ou la surveillance comprennent l'identification du début et/ou de l'exacerbation d'une bronchopneumopathie obstructive chronique chez le sujet sur la base du premier module et/ou du second module.

7. Procédé selon la revendication 6, dans lequel le diagnostic et/ou la surveillance comprennent l'identification du début de la bronchopneumopathie obstructive chronique chez le sujet sur la base à la fois de la valeur du premier module et de la valeur du second module.

8. Procédé selon la revendication 6 ou 7, dans lequel l'identification du début et/ou de l'exacerbation de la bronchopneumopathie obstructive chronique chez le sujet comprend l'identification d'une bronchopneumopathie obstructive chronique d'origine bactérienne sur la base d'une augmentation du second module.

9. Procédé selon la revendication 6, dans lequel le diagnostic et/ou la surveillance comprennent l'évaluation de l'efficacité d'un traitement de la bronchopneumopathie obstructive chronique chez le sujet sur la base à la fois de la valeur du premier module et de la valeur du second module.
